# EUROPÄISCHE PATENTSCHRIFT

(11) **EP 0 791 352 B1**
(45) Veröffentlichungstag und Bekanntmachung des Hinweises auf die Patenterteilung: **08.09.1999**
(21) Anmeldenummer: 97102489.8
(22) Anmeldetag: 15.02.1997
(51) Int. Cl.: A61K 7/13

(54) **Haarfärbemittel**
Hair dyeing composition
Composition pour la teinture des cheveux

(30) Priorität: 24.02.1996 DE 19606976; 29.05.1996 DE 19621499; 29.05.1996 DE 19621497
(43) Veröffentlichungstag der Anmeldung: 27.08.1997
(73) Patentinhaber: GOLDWELL GmbH, D-64297 Darmstadt (DE)
(72) Erfinder: Lorenz, Heribert, 64401 Gross-Bieberau (DE); Golinski, Frank, Dr., 64297 Darmstadt (DE); Kufner, Frank, 64297 Darmstadt (DE)

(56) Entgegenhaltungen:
- EP-A- 0 634 163
- EP-A- 0 634 164
- EP-A- 0 667 143

## Beschreibung

Die vorliegende Erfindung betrifft ein Haarfärbemittel auf Basis eines mit Peroxid reagierenden Entwickler-Kuppler-Systems, das einen ausdrucksvollen, brillanten und dauerhaften roten bzw. rotvioletten Grundton liefert, der entweder als solcher angewandt, oder, in Kombination mit weiteren Entwickler- und/oder Kupplersubstanzen, zur Erzielung zusätzlicher Farbnuancen benutzt werden kann.

Die nach wie vor in Haarfärbemitteln meist eingesetzten Entwicklersubstanzen sind 1,4-Diaminobenzol (p-Phenylendiamin) und 1-Methyl-2,5-diaminobenzol (p-Toluylendiamin). Die Verwendung dieser Substanzen ist insofern nicht völlig problemfrei, als sie bei extrem empfindlichen Personen in speziellen Fällen zu Hautsensibilisierungen führen können (bei sogenannten "Para-Allergikern").

Es wurde bereits versucht, dieses Problem durch Verwendung alternativer Entwicklersubstanzen zu lösen. Dies ist in beschränktem Umfang möglich durch den Einsatz von Tetraaminopyrimidin oder 2-(2,5-Diaminophenyl)ethanol (vgl. EP-A 7537 und EP-B 400 330); jedoch müssen dann erhebliche Abstriche in der Färbeintensität und den Variationsmöglichkeiten der verschiedenen Farbtöne hingenommen werden.

Aus der EP-A 0 634 164 sind Haarfärbemittel bekannt, die ein Kombination aus 2-Methyl-5-aminophenol, mindestens einer Verbindung aus der Gruppe 3-Methyl-4-aminophenol, 2-Methyl-4-aminophenol und 2-Hydroxymethyl-4-aminophenol sowie einem p-Phenylendiamin bzw. Bisphenylalkylendiaminderivat enthalten.

Eine weitgehend optimale Lösung des Problems, nämlich die Abwesenheit von Hautsensibilisierung einerseits und eine große Variationsbreite der Erzielung möglicher Farbnuancen andererseits, wird durch den in der EP-A 615 743 beschriebenen Einsatz von 2-(2'-Hydroxyethylamino)-5-aminotoluol bzw. dessen wasserlöslichen Salzen als Entwicklersubstanzen in Haarfärbemitteln erreicht.

Auch 1-Amino-4-bis-(2'-hydroxyethyl)aminobenzol (N,N-Bis-(2-hydroxyethyl)-p-phenylendiamin) und dessen Salze sind bereits als Entwicklersubstanzen in Haarfärbemitteln vorgeschlagen worden und in der vorläufigen Zulassungsliste für Haarfarben der EG unter der Nr. A 50 enthalten; jedoch bleiben auch bei Verwendung dieser Substanzen in Kombination mit den üblichen Kupplersubstanzen noch farbtechnische Wünsche offen.

Die Erfindung geht daher von der Aufgabenstellung aus, ein Haarfärbemittel zu schaffen, das 2-(2'-Hydroxyethylamino)-5-aminotoluol, 1-Amino-4-bis(2 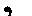 -hydroxyethyl)aminobenzol und/oder 2-(2,5-Diaminophenyl)ethanol bzw. deren wasserlösliche Salze als Entwicklersubstanz(en) enthält und zur Herstellung eines roten bzw. rotvioletten Farbtons geeignet ist.

Diese Aufgabe wird einmal dadurch gelöst, daß ein solches Haarfärbemittel ein mit Peroxid reagierendes Entwickler-Kuppler-System enthält, das aus einer Kombination aus 2-(2'-Hydroxyethylamino)-5-aminotoluol und/oder 1-Amino-4-bis-(2 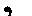 -hydroxyethyl)aminobenzol bzw. deren wasserlöslichen Salzen, 5-Amino-2-methylphenol und 4-Amino-3-methylphenol besteht.

Bei Anwendung dieser Zusammensetzungen auf Basis einer üblichen Grundlage wird nach der Oxidation mit Peroxid eine sehr ausdrucksvolle, intensive rote bzw. rotviolette Grundfärbung erhalten, die durch Zusatz entsprechender weiterer Kupplersubstanzen zu anderen Farbnuancen variiert werden kann.
Solche bevorzugten weiteren Kupplersubstanzen sind insbesondere Resorcin, 2-Methylresorcin, 4-Chlorresorcin, 3-Aminophenol, 2-Aminophenol, 2-Amino-4-β-hydroxyethylaminoanisol bzw. dessen wasserlösliche Salze und α-Naphthol.
Damit soll jedoch der Zusatz weiterer Kupplersubstanzen keineswegs ausgeschlossen sein. Die Aufgabenlösung besteht weiterhin darin, daß ein erfindungsgemäßes Haarfärbemittel eine Kombination aus
a) 2-(2,5-Diaminophenyl)ethanol bzw. dessen wasserlöslichen Salzen;
b) 5-Amino-2-methylphenol;
c) 4-Amino-3-methylphenol; und
d) mindestens einer Kupplersubstanz, aus gewählt aus der Gruppe Resorcin, 2-Methylresorcin, 4-Clorresorcin, 3-Aminophenol, α-Naphthol, 3-Amino-2-methylamino-6-methoxypyridin und/oder 2-Amino-4-(β-hydroxyethylamino)anisol bzw. dessen wasserlöslichen Salzen, enthält.

Auch die Mitverwendung weiterer, an sich bekannter Entwicklersubstanzen ist möglich. Neben den bereits eingangs genannten sind hierbei insbesondere noch 4-Aminophenol, 5-Aminoalicylsäure und/oder Hydroxytriaminopyrimidine, wie sie aus der EP-B 467 026 bekannt sind, zu erwähnen.
Als solche werden insbesondere das aus der bereits erwähnten EP-B 467 026 bekannte 2,4,5-Triamino-6-hydroxypyrimidin und das 4,5,6-Triamino-2-hydroxypyrimidin eingesetzt, insbesondere auch als Sulfat-Salze.

Die Konzentration der Entwicklersubstanzen liegt zwischen etwa 0,05 und 5%, vorzugsweise 0,1 und 4%, insbesondere 0,25 bis 0,5% und 2,5 bis 3% Gew.-% der Gesamtzusammensetzung des Haarfärbemittels (ohne Oxidationsmittel), wobei sich die Angaben jeweils auf den Anteil an freier Base beziehen.

Die Kupplersubstanzen als Reaktionspartner der Entwicklersubstanz(en) liegen in den erfindungsgemäßen Haafärbemitteln etwa im gleichen Anteil wie die Entwicklersubstanzen vor, d. h., also in Mengen von 0,05 bis 5,0%, vorzugsweise 0,1 bis 4%, insbesondere 0,5 bis 3 Gew.-% der Gesamtzusammensetzung (ohne Oxidationsmittel), wobei sich die Angaben jeweils auf den Anteil an freier Base beziehen.

Auch hier ist es, wie bereits erwähnt, möglich und zuweilen auch zweckmäßig, weitere bekannte Kupplersubstanzen mitzuverwenden, falls dies zur Erzielung bestimmter Farbnuancen erwünscht und erforderlich ist.

Die erfindungsgemäßen Zusammensetzungen können erwünschtenfalls auch sogenannte Nuanceure zur Feineinstellung des gewünschten Farbtones, insbesondere auch direktziehende Farbstoffe, enthalten.

Solche Nuanceure sind beispielsweise Nitrofarbstoffe wie 2-Amino-4,6-dinitrophenol, 2-Amino-4-nitrophenol, 2-Amino-6-chlor-4-nitrophenol, etc., vorzugsweise in Mengen von etwa 0,05 bis 2,5%, insbesondere 0,1 bis 1% Gew.-% der Farbzusammensetzung (ohne Oxidationsmittel).

Die erfindungsgemäßen Haafärbemitteln können die in solchen Mitteln üblichen Grund-und Zusatzstoffe, Konditioniermittel, etc. enthalten, die dem Fachmann aus dem Stand der Technik bekannt und beispielsweise in der Monographie von K. Schrader, "Grundlagen und Rezepturen der Kosmetika", 2. Aufl. (Hüthig Buch Verlag, Heidelberg, 1989), S. 782 bis 815, beschrieben sind. Sie können als Lösungen, Cremes, Gele oder auch in Form von Aerosol-Präparaten vorliegen; geeignete Trägermaterial-Zusammensetzungen sind aus dem Stand der Technik hinreichend bekannt.

Zur Applikation wird das erfindungsgemäße Oxidationsfarbstoff-Vorprodukt mit einem Oxidationsmittel vermischt. Bevorzugtes Oxidationsmittel ist Wasserstoffperoxid, beispielsweise in 2- bis 6-prozentiger Konzentration.

Es können jedoch auch andere Peroxide wie Harnstoffperoxid und Melaminoperoxid eingesetzt werden.

Der pH-Wert des applikationsfertigen Haafärbemittels, d. h. nach Vermischung mit Peroxid, kann sowohl im schwach sauren, d. h. einem Bereich von 5,5 bis 6,9, im neutralen als auch im alkalischen Bereich, d. h. zwischen pH 7,1 und 9,5 liegen.

Im folgenden werden verschiedene Ausführungsbeispiele zur Erläuterung der Erfindung gegeben.

### Grundlage

| | |
|---|---|
| Stearylalkohol | 8,0 (Gew.-%) |
| Kokosfettsäuremonoethanolamid | 4,5 |
| 1,2-Propandiolmono/distearat | 1,3 |
| Kokosfettalkoholpolyglykolether | 4,0 |
| Natriumlaurylsulfat | 1,0 |
| Ölsäure | 2,0 |
| 1,2-Propandiol | 1,5 |
| Na-EDTA | 0,5 |
| Natriumsulfit | 1,0 |
| Eiweißhydrolysat | 0,5 |
| Ascorbinsäure | 0,2 |
| Parfüm | 0,4 |
| Ammoniak, 25%ig | 8,5 |
| Ammoniumchlorid | 0,5 |
| Panthenol | 0,8 |
| Wasser | @100,00 |

Die erfindungsgemäßen Entwickler-Kuppler-Kombinationen wurden jeweils, unter entsprechender Verringerung des Wassergehalts, in diese Grundlage eingearbeitet.

Die Ausfärbungen erfolgten jeweils an Woll-Läppchen und Strähnen aus gebleichtem Menschenhaar, durch Aufbringen einer 1:1-Mischung aus Farbstoff-Vorprodukt und 6%iger Wasserstoffperoxid-Lösung und zwanzigminütiger Einwirkung bei Zimmertemperatur, folgendem Auswaschen und Trocknen.

Es wurden die folgenden Färbungen erzielt:
Beispiel 1:

| | |
|---|---|
| 0,36 (Gew.-%) | 2-(2'-Hydroxyethylamino)-5-aminotoluolsulfat (HAT) |
| 0,14 | 4-Amino-3-methylphenol |
| 0,28 | 5-Amino-2-methylphenol |

Färbung: Kräftiges Rotviolett (Graumagenta).
Beispiel 1a: Weglassen von 4-Amino-3-methylphenol führte zu einer blauvioletten, nicht verwertbaren Färbung.
Beispiel 2:

| | |
|---|---|
| 0,36 (Gew.-%) | HAT |
| 0,14 | 4-Amino-3-methylphenol |
| 0,10 | 5-Amino-2-methylphenol |
| 0,10 | 3-Aminophenol |
| 0,10 | Resorcin |

Färbung: Graurubin.
Beispiel 3:

| | |
|---|---|
| 0,36 (Gew.-%) | HAT |
| 0,14 | 4-Amino-3-methylphenol |
| 0,14 | 5-Amino-2-methylphenol |
| 0,31 | 2-Amino-4-(2'-hydroxyethylamino)anisolsulfat |

Färbung: Dunkelgrauviolett.
Beispiel 4:

| | |
|---|---|
| 0,36 (Gew.-%) | HAT |
| 0,14 | 4-Amino-3-methylphenol |
| 0,14 | 5-Amino-2-methylphenol |
| 0,25 | 3-Amino-2-methylamino-6-methoxypyridinsulfat |

Färbung: Grauviolett.
Beispiel 5:

| | |
|---|---|
| 0,36 (Gew.-%) | HAT |
| 0,14 | 4-Amino-3-methylphenol |
| 0,14 | 5-Amino-2-methylphenol |
| 0,16 | α-Naphthol |

Färbung: Graumagenta (rotviolett).
Beispiel 6:

| | |
|---|---|
| 0,33 (Gew.-%) | 1-Amino-4-bis-(2'-hydroxyethyl)aminobenzolsulfat (A 50) |
| 0,14 | 4-Amino-3-methylphenol |
| 0,28 | 5-Amino-2-methylphenol |

Färbung: Kräftiges Blauviolett.
Beispiel 6a: Weglassen von 4-Amino-3-methylphenol führte zu einer rotstichigen, nicht verwertbaren Blaufärbung.
Beispiel 7:

| | |
|---|---|
| 0,36 (Gew.-%) | A 50 |
| 0,14 | 4-Amino-3-methylphenol |
| 0,10 | 5-Amino-2-methylphenol |
| 0,08 | 3-Aminophenol |
| 0,08 | Resorcin |

Färbung: Intensives Grauviolett.
Beispiel 8:

| | |
|---|---|
| 0,33 (Gew.%) | A 50 |
| 0,14 | 4-Amino-3-methylphenol |
| 0,14 | 5-Amino-2-methylphenol |
| 0,31 | 2-Amino-4-(2'-hydroxyethylamino)anisolsulfat |

Färbung: Intensives Graublau.
Beispiel 9:

| | |
|---|---|
| 0,33 (Gew.-%) | A 50 |
| 0,14 | 4-Amino-3-methylphenol |
| 0,14 | 5-Amino-2-methylphenol |
| 0,26 | 3-Amino-2-methylamino-6-methoxypyridinsulfat |

Färbung: Kräftiges Rauchblau.
Beispiel 10:

| | |
|---|---|
| 0,33 (Gew.-%) | A 50 |
| 0,14 | 4-Amino-3-methylphenol |
| 0,14 | 5-Amino-2-methylphenol |
| 0,16 | α-Naphthol |

Färbung: Intensives Grauviolett.
Beispiel 11:

| | |
|---|---|
| 0,28 (Gew.-%) | 1-(β-Hydroxyethyl)-2,5-diaminobenzolsulfat (A 80)* |
| 0,14 | 4-Amino-3-methylphenol |
| 0,28 | 5-Amino-2-methylphenol |

| | |
|---|---|
| * nach der vorgeschlagenen Positivliste für Oxidationshaarfarbstoffe der EG. | |

Färbung: Kräftiges Rotviolett (Graumagenta).
Beispiel 11a: Weglassen von 4-Amino-3-methylphenol führte zu einer beigebraunen, nicht verwertbaren Färbung.
Beispiel 12:

| | |
|---|---|
| 0,36 (Gew.-%) | A 80 |
| 0,14 | 4-Amino-3-methylphenol |
| 0,10 | 5-Amino-2-methylphenol |
| 0,09 | 3-Aminophenol |
| 0,09 | Resorcin |

Färbung: Intensives Rotbraun.
Beispiel 13:

| | |
|---|---|
| 0,28 (Gew.-%) | A 80 |
| 0,14 | 4-Amino-3-methylphenol |
| 0,14 | 5-Amino-2-methylphenol |
| 0,31 | 2-Amino-4-(2'-hydroxyethylamino)anisolsulfat |

Färbung: Intensives Grauviolett.
Beispiel 14:

| | |
|---|---|
| 0,28 (Gew.-%) | A 80 |
| 0,14 | 4-Amino-3-methylphenol |
| 0,14 | 5-Amino-2-methylphenol |
| 0,25 | 3-Amino-2-methylamino-6-methoxypyridinchlorid |

Färbung: Kräftiges Graublau.
Beispiel 15:

| | |
|---|---|
| 0,28 (Gew.-%) | A 80 |
| 0,14 | 4-Amino-3-methylphenol |
| 0,14 | 5-Amino-2-methylphenol |
| 0,16 | α-Naphthol |

Färbung: Graumagenta (rotviolett).

Die Farbbezeichnungen richten sich nach der Einteilung des "Taschenlexikons der Farben", 3. Aufl. (1981, Muster-Schmidt Verlag, Zürich - Göttingen).

## Patentansprüche

1. Haarfärbemittel auf Basis eines mit Peroxid reagierenden Entwickler-Kuppler-Systems, dadurch gekennzeichnet, daß es eine Kombination aus
a) mindestens einer Entwicklersubstanz, ausgewählt aus 2-(2 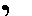 -Hydroxyethylamino)-5-aminotoluol, und/oder 1 -Amino-4-bis-(2 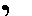 -hydroxyethyl)aminobenzol bzw. deren wasserlöslichen Salzen;
b) 5-Amino-2-methylphenol; und
c) 4-Amino-3-methylphenol
enthält.

2. Haarfärbemittel nach Anspruch 1, dadurch gekennzeichnet, daß es mindestens eine zusätzliche Kupplersubstanz, ausgewählt aus der Gruppe Resorcin, 2-Methylresorcin, 4-Chlorresorcin, 3-Aminophenol, α-Naphthol, 3-Amino-2-methylamino-6-methoxypyridin und/oder 2-Amino-4-(β-hydroxyethylamino)anisol bzw. dessen wasserlöslichen Salzen, enthält.

3. Haarfärbemittel auf Basis eines mit Peroxid reagierenden Entwickler-Kuppler-Systems, dadurch gekennzeichnet, daß es eine Kombination aus
a) 2-(2,5-Diaminophenyl)ethanol bzw. dessen wasserlöslichen Salzen;
b) 5-Amino-2-methylphenol;
c) 4-Amino-3-methylphenol; und
d) mindestens einer Kupplersubstanz, aus gewählt aus der Gruppe Resorcin, 2-Methylresorcin, 4-Clorresorcin, 3-Aminophenol, α-Naphthol, 3-Amino-2-methylamino-6-methoxypyridin und/oder 2-Amino-4-(β-hydroxyethylamino)anisol bzw. dessen wasserlöslichen Salzen, enthält.

## Claims

1. Hair dyeing composition, based on a developer/coupler system reacting with peroxides, characterized in that it comprises a combination of
a) at least one developing compound selected from 2-(2,5-hydroxyethyl amino)-5-aminotoluene and (or) 1-amino-4-bis-(2'-hydroxyethyl amino) aminobenzene and(or)the water-soluble salts thereof, resp.;
b) 5-amino-2-methyl phenol; and
c) 4-amino-3-methyl phenol.

2. Hair dyeing composition according to claim 1 characterized in that it comprises at least one additional coupling substance, selected from the group resorcinol, 2-methyl resorcinol, 4-chlororesorcinol, 3-aminophenol, α-naphthol, 3-amino-2-methyl amino-6-methoxypyridine and (or) 2-amino-4-(β-hydroxyethyl amino) anisole, and the water-soluble salts thereof, resp.

3. Hair dyeing composition, based on a developer-coupler-system reacting with peroxide, characterized in that it comprises a combination of
a) 2-(2.5-diaminophenol)ethanol and its water-soluble salts, resp.;
b) 5-amino-2-methyl phenol;
c) 4-amino-3-methyl phenol; and
d) at least one coupling substance, selected from the group resorcinol, 2-methyl resorcinol, 4-chlororesorcinol, 3-aminophenol, α-naphthol, 3-amino-2-methyl amino-6-methoxypyridine and (or) 2-amino-4-(β-hydroxyethyl amino) anisole, and the water-soluble salts thereof.

## Revendications

1. Composition tinctoriale pour des cheveux à la base d'un système dévoloppeur-coupleur réagissant avec des peroxydes, caractérisée par le fait qu'elle comprend une combinaison
a) d'au moins d'une substance développement, sélectionnée parmi la 2-(2'-hydroxyéthylamino)-5-aminotoluène et (ou) la 1-amino-4-bis-(2'-hydroxyéthyl)aminobenzène et leur sels hydrosolubles;
b) 5-amino-2-méthylphénol; et
c) 4-amino-3-méthylphénol.

2. Composition selon la revendication 1, caractérisée par le fait qu'elle contient au moins d'une substance coupleur additionnelle sélectionnée parmi le groupe résorcine, 2-méthylrésorcine, 4-chlororésorcine, 3-aminophénol, α-naphthol, 3-amino-2-méthylamino-6-méthoxypyridine et (ou) 2-amino-4-(β-hydroxyéthylamino) anisole ou leur sels hydrosolubles.

3. Composition tinctoriale pour des cheveux à la base d'un système dévolloppeur-coupleur réagissant avec des peroxydes, caractérisée par le fait qu'elle comprend une combinaison
a) du 2-(2,5-diaminophényl)éthanol et son sels hydrosolubles;
b) 5-amino-2-méthylphénol;
c) 4-amino-3-méthylphénol; et
d) d'au moins d'une substance coupleur, sélectionnée parmi le groupe résorcine, 2-méthylrésorcine, 4-chlororésorcine, 3-aminophénol, α-naphthol, 3-amino-2-méthylamino-6-méthoxypyridine et (ou) 2-amino-4-(β-hydroxyéthylamino) anisole ou leur sels hydrosolubles.
